# EUROPEAN PATENT SPECIFICATION

(11) **EP 3 805 746 B1**
(45) Date of publication and mention of the grant of the patent: **07.06.2023**
(21) Application number: 19811439.9
(22) Date of filing: 29.05.2019
(51) Int. Cl.: G01N 27/414, G01N 27/30, G01N 33/68, H01L 21/027, G01N 33/543

(54) **MANUFACTURING METHOD OF RGO-BASED BIOSENSOR**
VERFAHREN ZUR HERSTELLUNG EINES BIOSENSOR AUF RGO-BASIS
PROCÉDÉ DE FABRICATION D'UN BIOCAPTEUR À BASE DE RGO

(30) Priority: 31.05.2018 KR 20180062487
(43) Date of publication of application: 14.04.2021
(73) Proprietor: XYZ Platform Inc., Seoul 04783 (KR)
(72) Inventor: HWANG, Kyo Seon, Seoul 02799 (KR); CHAE, Myung Sic, Seoul 08781 (KR); PARK, Dong Sung, Seoul 06313 (KR); YOO, Yong Kyoung, Seoul 01820 (KR); KIM, Jae Hyun, Seoul 02804 (KR)
(74) Representative: Walaski, Jan Filip
(86) International application number: PCT/KR2019/006414
(87) International publication number: WO 2019/231224

(56) References cited:
- KR-B1- 101 400 976
- KR-B1- 101 430 140
- CHAN CHUNYU ET AL: "A microfluidic flow-through chip integrated with reduced graphene oxide transistor for influenza virus gene detection", SENSORS AND ACTUATORS B: CHEMICAL, ELSEVIER BV, NL, vol. 251, 26 May 2017 (2017-05-26), pages 927-933, XP085254172, ISSN: 0925-4005, DOI: 10.1016/J.SNB.2017.05.147
- RONG XIANG HE ET AL: "Solution-Gated Graphene Field Effect Transistors Integrated in Microfluidic Systems and Used for Flow Velocity Detection", NANO LETTERS, vol. 12, no. 3, 14 March 2012 (2012-03-14) , pages 1404-1409, XP055043754, ISSN: 1530-6984, DOI: 10.1021/nl2040805
- XU SHICAI ET AL: "Ultrasensitive label-free detection of DNA hybridization by sapphire-based graphene field-effect transistor biosensor", APPLIED SURFACE SCIENCE, vol. 427, 14 September 2017 (2017-09-14), pages 1114-1119, XP085282929, ISSN: 0169-4332, DOI: 10.1016/J.APSUSC.2017.09.113
- GUANGFU WU ET AL: "Graphene Field-Effect Transistors for the Sensitive and Selective Detection of Escherichia coli Using Pyrene-Tagged DNA Aptamer (Supporting Information)", ADVANCED HEALTHCARE MATERIALS, vol. 6, no. 19, 10 August 2017 (2017-08-10), pages 1-22, XP055584661, DE ISSN: 2192-2640, DOI: 10.1002/adhm.201700736
- IL-YUNG SOHN ET AL: "pH sensing characteristics and biosensing application of solution-gated reduced graphene oxide field-effect transistors", BIOSENSORS AND BIOELECTRONICS, vol. 45, 4 February 2013 (2013-02-04), pages 70-76, XP055493268, Amsterdam , NL ISSN: 0956-5663, DOI: 10.1016/j.bios.2013.01.051
- WU et al.: "Graphene Field-Effect Transistors for the Sensitive and Selective Detection of Escherichia coli Using Pyrene-Tagged DNA Aptamer", Advanced Healthcare Materials, vol. 6, no. 19, 1 October 2017 (2017-10-01), pages 1-22, XP055584661, ISSN: 2192-2640, DOI: 10.1002/adhm.201700736
- HE et al.: "Solution-Gated Graphene Field Effect Transistors Integrated in Microfluidic Systems and Used for Flow Velocity Detection", Nano Letters, vol. 12, no. 3, 14 March 2012 (2012-03-14) , pages 1404-1409, XP055043754, ISSN: 1530-6984, DOI: 10.1021/nl2040805
- Wu, Guangfu et al.: "Graphene Field-Effect Transistors for the Sensitive and Selective Detection of Escherichia coli Using Pyrene-Tagged DNA Aptamer (Supporting Information)", Graphene Field-Effect Transistors for the Sensitive and Selective Detection of Escherichia coli Using Pyrene-Tagged DNA Aptamer (Supporting Information), vol. 6, no. 19, 10 August 2017 (2017-08-10), pages 1-21, XP055584661, DOI: 10.1002/adhm.201700736

## Description

### TECHNICAL FIELD

The present invention relates to a manufacturing method for a RGO (reduced graphene oxide) based biosensor.

### BACKGROUND OF THE INVENTION

Graphene is a nano material having an excellent physical property for which various researches are being waged in order to obtain economic feasibility and performances for application to electric elements and sensors. Meantime, complex processes and high-priced equipment are required to obtain a pure monolayered graphene having the best electrical and physical characteristics, disallowing to have a device manufacturing of the graphene through mass production and resultantly leading to the decreased economic feasibility. GOs (Graphene Oxides) obtainable by applying chemical processes onto graphite include an oxygen reactor to thereby allow being easily soluble in polar solvent like water and applying various low-cost deposition techniques based on solution processes using the RGO. Although an RGO film including an oxygen reactor, unlike a pure graphene, is an insulator, the RGO through a chemical reduction reaction can obtain an electrical conductivity to allow being applicable as an electric device.

Meantime, in light of the fact that most of the biochemical reaction is realized from liquid phase, a bio sensor capable of driving within a liquid phase must be developed in order to perform a real-time detection of target material. Particularly, because a bio sensor in the form of FET has a high sensitivity and structural simplicity, the bio sensor is widely researched as a bio sensor.

However, the FET generally suffers from a disadvantage of requiring a high driving voltage because of a back-gated FET structure having a high-k material as a gate material. Furthermore, several problems arise when a bio sensor is driven in a biochemical reaction occurring liquid phase and applied at a site. For example, it is not easy to expose only an RGO pattern in a reaction solution, and it is also difficult to attach a micro channel by being matched to the RGO pattern.

The use of graphene field effect transistors in biosensors is documented in:
CHAN CHUNYU ET AL: "A microfluidic flow-through chip integrated with reduced graphene oxide transistor for influenza virus gene detection", SENSORS AND ACTUATORS B: CHEMICAL, ELSEVIER BV, NL, vol. 251, 26 May 2017 (2017-05-26), pages 927-933;
RONG XIANG HE ET AL: "Solution-Gated Graphene Field Effect Transistors Integrated in Microfluidic Systems and Used for Flow Velocity Detection", NANO LETTERS, vol. 12, no. 3, 14 March 2012 (2012-03-14), pages 1404-1409;
XU SHICAI ET AL: "Ultrasensitive label-free detection of DNA hybridization by sapphire-based graphene field-effect transistor biosensor", APPLIED SURFACE SCIENCE, vol. 427, 14 September 2017 (2017-09-14), pages 1114-1119;
GUANGFU WU ET AL: "Graphene Field-Effect Transistors for the Sensitive and Selective Detection of Escherichia coli Using Pyrene-Tagged DNA Aptamer (Supporting Information)", ADVANCED HEALTHCARE MATERIALS, vol. 6, no. 19, 10 August 2017 (2017-08-10), pages 1-22; and
IL-YLTNG SOHN ET AL: "pH sensing characteristics and biosensing application of solution-gated reduced graphene oxide field-effect transistors", BIOSENSORS AND BIOELECTRONICS, vol. 45,4 February 2013 (2013-02-04), pages 70-76.

### DETAILED DESCRIPTION OF THE INVENTION

### TECHNICAL SUBJECT

The present invention is provided to solve the aforementioned disadvantages.

### TECHNICAL SOLUTION

According to the invention, there is provided a method for manufacturing a RGO (Reduced Graphene Oxide) based biosensor according to claim 1. Preferred features are set out in the dependent claims.

### ADVANTAGEOUS EFFECTS

The bio sensor according to the present invention includes a passivation layer where the passivation layer may perform the sealing less a reaction solution supplied to an RGO channel through a PDMS chip be leaked to outside including a source electrode and a drain electrode. As a result, a bio material can be accurately detected without any error.

Particularly, the passivation layer may be formed with SU-8 photoresist, where the SU-8 photoresist, unlike general photoresist (PR) polymers, has bio-friendliness level during curing process, and has also fire resistance (flame retardant characteristic) against polar solvent acid/base, and therefore, the SU-8 photoresist has a physical property adequate for solution exposure for biochemical reaction.

Furthermore, the gate voltage may be directly applied to a reaction solution while the reaction solution is contacted to the RGO channel, through which a FET type bio sensor having a high sensitivity drivable at a gate voltage of -1.0V ~ 1.0V can be realized.

### BRIEF DESCRIPTION OF THE DRAWINGS

FIG. 1 is an RGO-based bio sensor resulting from the method according to the present invention.
FIG. 2 is a method for manufacturing an RGO-based bio sensor according to an embodiment of the present invention.
FIG. 3 is an example of explaining each process for manufacturing an RGO-based bio sensor.
FIG. 4 is an example for XPS analysis of RGO film.
FIG. 5 is an example of a real product of RGO-based bio sensor not according to the present invention.
FIG. 6 is a method for detecting a bio material using an RGO-based bio **sensor** which was manufactured according to the present invention.
FIG. 7 is an example for explaining a Dirac point.
FIG. 8 is an example related to an experiment for quantitively detecting Aβ protein which is a bio marker for diagnosing an Alzheimer's disease.
FIG. 9 is an example of ion sensitivity observation experiment using an RGO-based bio sensor.
FIG. 10 is an example of AChE enzyme reaction detection experiment using an RGO-based bio sensor.
FIG. 11 is an example of drug-effect observation experiment using AChE inhibitor.

### BEST MODE

The present invention may be applied with various changes and have several exemplary embodiments, where particular exemplary embodiments will be exemplified in the drawings and will be described in detail through the detailed description of the present invention.

In describing the present invention, detailed descriptions of well-known technologies are omitted for brevity and clarity so as not to obscure the description of the present invention with unnecessary detail. The terminology used herein is for the purpose of describing particular implementations only and is not intended to be limiting of the invention. As used herein, the singular forms are intended to include the plural forms as well, unless the context clearly indicates otherwise.

It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

It will be understood that, although the terms first, second, etc. may be used herein to describe various elements, these elements should not be limited by these terms. These terms are only used to distinguish one element from another.

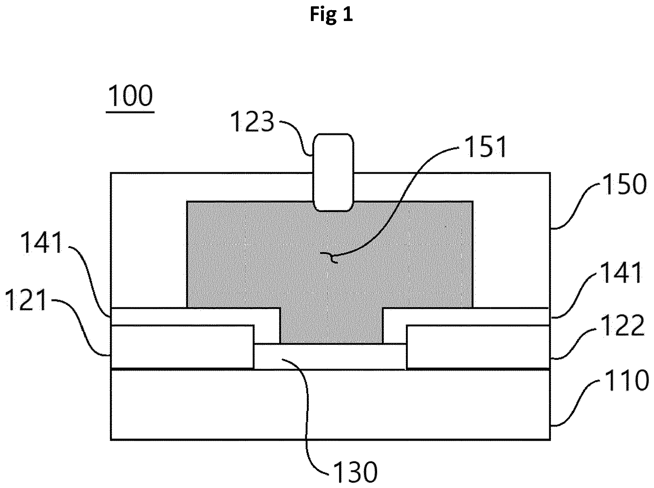

Referring to FIG. 1, an RGO-based bio sensor (100) resulting from the method of the present invention may be formed on a substrate (110), where the substrate (110) may be formed with a source electrode (121) and a drain electrode (122), and an RGO (Reduced Graphene Oxide) channel (130) may be formed between the source electrode (121) and the drain electrode (122).

The substrate (110) may be formed with various materials. In a detailed example, although the substrate (110) may be used with a substrate in which SiO2 is deposited on a silicone (Si) substrate, the present invention is not limited thereto. The source electrode (121) and the drain electrode (122) may be formed with various materials, and may be formed with chrome and gold (Cr/Au), but the present invention is not limited thereto.

A passivation layer (141) is formed on the source electrode (121), the drain electrode (122) and the RGO channel (130), where the passivation layer (141) may perform a role of preventing a reaction solution supplied through a PDMS chip (150) from being brought into contact with the source electrode (121) and the drain electrode (122). That is, the passivation layer (141) may prevent the reaction solution from flowing to outside by sealing a region encompassing a portion of the RGO channel (130) that must be contacted to the reaction solution.

Toward this end, the passivation layer (141) may be preferably formed in consideration of not only the sealing function but also attaching function of PDMS(Polydimethylsiloxane) chip (150). One of the exemplary embodiments for forming the passivation layer (141) may be the formation of the passivation layer (141) with an SU-8 photoresist. When the passivation layer (141) is formed using an SU-8 photoresist, attachment with the PDMS chip (150) is possible by the Van der Waals force without recourse to a separate means for attaching the PDMS chip (150). Furthermore, an adhesive strength strong enough to prevent leakage or separation may be secured even in a simple attachment state during infusion of reaction solution or measurement in liquid phase.

The PDMS chip (150) is provided to supply a reaction solution to the RGO channel (130), and the PDMS chip (150) may be formed therein with a space (micro channel, 151) for supplying the reaction solution. The said micro channel (151) may be variably formed, and may be formed with an inlet through which a reaction solution is inputted and an outlet through which the reaction solution is discharged.

When the PDMS chip (150) is attached to an upper end of the passivation layer (141), a reaction solution is supplied through the micro channel (151) formed inside of the PDMS chip (150), and the reaction solution becomes to be in a state of being contacted with the RGO channel (130). At this time, the gate electrode (123) may be electrically connected to the reaction solution contacted to the RGO channel (130). That is, the gate electrode (123) may be directly contacted to the reaction solution to realize a liquid driving (operation). To this end, the PDMS chip (150) may be so configured as to directly contact the reaction solution.

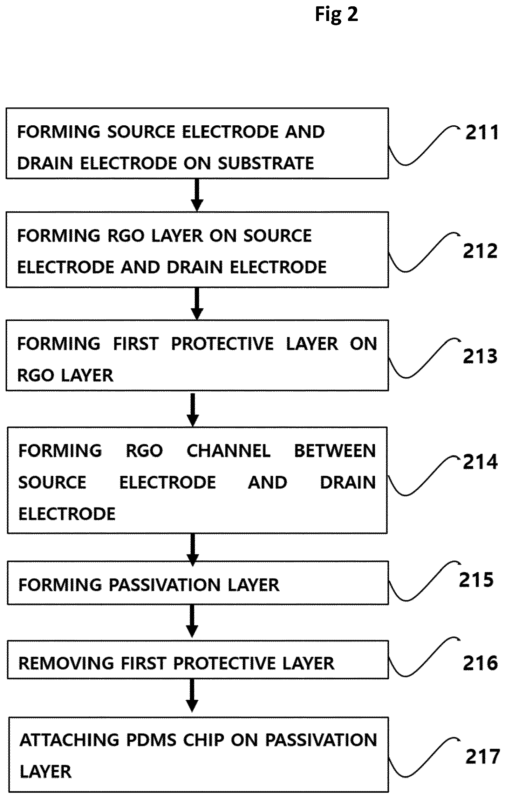

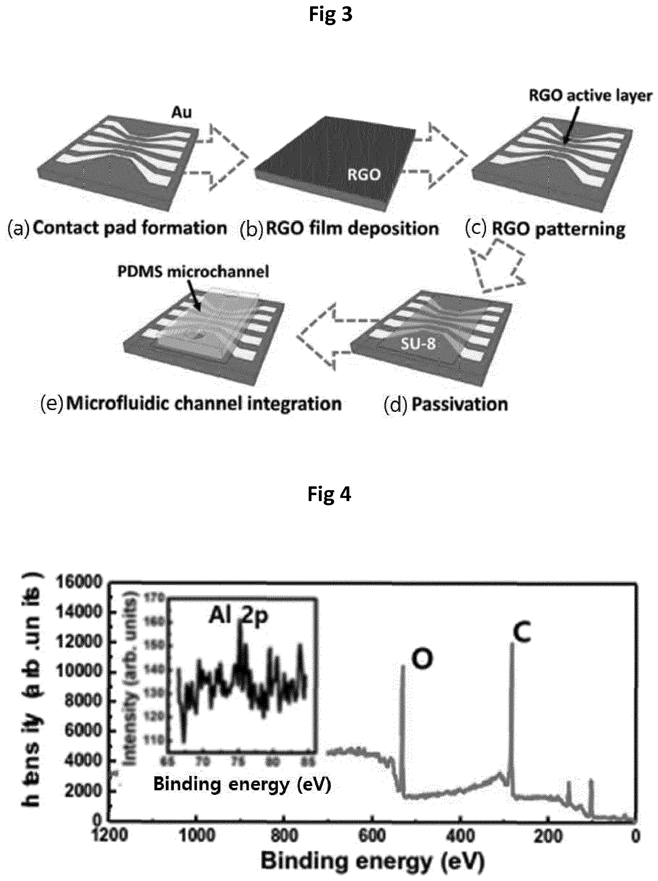

FIG. 2 is a method for manufacturing an RGO-based bio sensor according to an embodiment of the present invention, and FIG. 3 is a schematic view for showing a bio sensor manufacturing process.

Referring to FIGS. 2 and 3, a method for manufacturing an RGO-based bio sensor according to the present invention will be described in detail.

A first step: a source electrode and a drain electrode are formed on a substrate (211). The '211' step is preferably performed prior to formation of the RGO film. As a result, the RGO film may be prevented from being polluted in a subsequent process due to photoresist or organic materials. The method of forming the source electrode and the drain electrode in the '211' step may be variably realized. As a detailed example, the source electrode and the drain electrode may be formed by way of lift-off method in which patterning is performed using the photoresist, Cr/Au of 20nm/100nm is deposited on a front surface using an e-beam evaporator, which is then dipped in acetone to remove the photoresist.

FIG. 3a illustrates an example of a substrate on which the '211' step is performed.

A second step: an RGO layer is formed on the source electrode and the drain electrode (212). In order to deposit the RGO film, a GO (Graphene Oxide) is first formed by oxidizing graphic powder through 'Hummers' method. The GO may not be condensed in polar solvent like water to thereby allow being dispersed in solution form. In '212' step, in order to improve the binding force (coherence) between surface of graphene film and that of SiO₂, the 5% 3-aminopropyltriethoxysilane (APTES) may be fixated on SiO₂ for two hours prior to deposition. Then, the binding force may be enhanced by allowing an oxygen reactor of graphene to coalesce (covalent bond) in response to amine (amine, -NH2) reactor of APTES.

An oxidative graphene solution may be formed by diluting the GO in distilled water in the ratio of 3 mg/mL concentration, and spin coating is performed at '4000 rpm' for about '30 seconds' to allow being deposited on a 4-inch SiO₂ wafer. The deposited GO film lacks of electric conductivity and the RGO, which is a conductive material, may be formed by removing the oxygen reactor through reduction reaction (3b).

In a detailed example, the reduction reaction is accomplished by exposing the GO film in hydroiodic acid (HI) vapor of 80°C for about 3 hours.

A third step: A first protective layer is formed on an RGO layer (213).

The first protective layer is to prevent the pollution problem that may be generated when the RGO is patterned, and may be formed directly on the RGO film deposited in the second step.

In a detailed example, the first protective layer may be formed with Al₂O₃, where the Al₂O₃ layer may be formed with a thickness of about 50nm using an RF sputter.

A fourth step: an RGO channel is formed between a source electrode and a drain electrode by performing a patterning process (214).

The '214' step is a process of patterning an RGO active channel.

As a detailed example, in the '214' step, patterning may be performed on a deposited RGO/Al₂O₃ film through photoresist, and an RGO channel may be formed by a dry etching method using ICP-RIE (inductively coupled plasma reactive ion etching) and RIE equipment respectively (3c).

A fifth step: A passivation layer may be formed that encompasses a surrounding of an RGO channel including a source electrode and a drain electrode (215).

In order to allow an RGO-FET to smoothly drive in a liquid phase, only the RGO pattern is exposed to a solution, while the source electrode and the drain electrode must be insulated. This is because when the source electrode and the drain electrode are exposed to the reaction solution, a current leakage may be generated to gate-drain when a gate bias is applied to make it impossible to perform a normal FET operation.

The passivation layer may be formed by using SU-8 (SU8-2005) which is a negative photoresist known to be an insulator and to have biocompatibility. The SU-8 photoresistor, unlike the common photoresist polymer, has the biocompatibility at the time of curing process, and has a physical property adequate to solution exposure for biochemical reaction because of having fire-resistance ((flame retardant characteristic) to polar solution or acid/base. As a detailed example for forming a passivation layer, SU-8 may be coated, by way of spin coating, on a front surface of wafer formed with the source electrode, drain electrode and RGO/Al₂O₃ pattern, and patterning may be performed through photolithography (3d).

A sixth step: A first protective layer is removed (216). That is, the first protective layer (Al₂O₃) is removed to expose only the RGO channel to the reaction solution. The removal of the first protective layer may be variably performed. In a detailed example, the removal of Al₂O₃ may be performed under an environment in which a wet etchant (ceramic etchant A, sigma-aldrich) having a high selectivity is heated at a temperature of 180°C, through which only the Al₂O₃ can be selectively removed, and it can be ascertained from FIG. 4 that no residual Al remains on the graphene through XPS.

A seventh step: The passivation layer may be now attached thereon with a PDMS chip for supplying a reaction solution to the RGO channel (217). When the passivation layer is formed using the SU-8 photoresist, the PDMS chip may be attached onto the passivation layer without a separate means for attaching the PDMS chip by using Van der Waals force (3e).

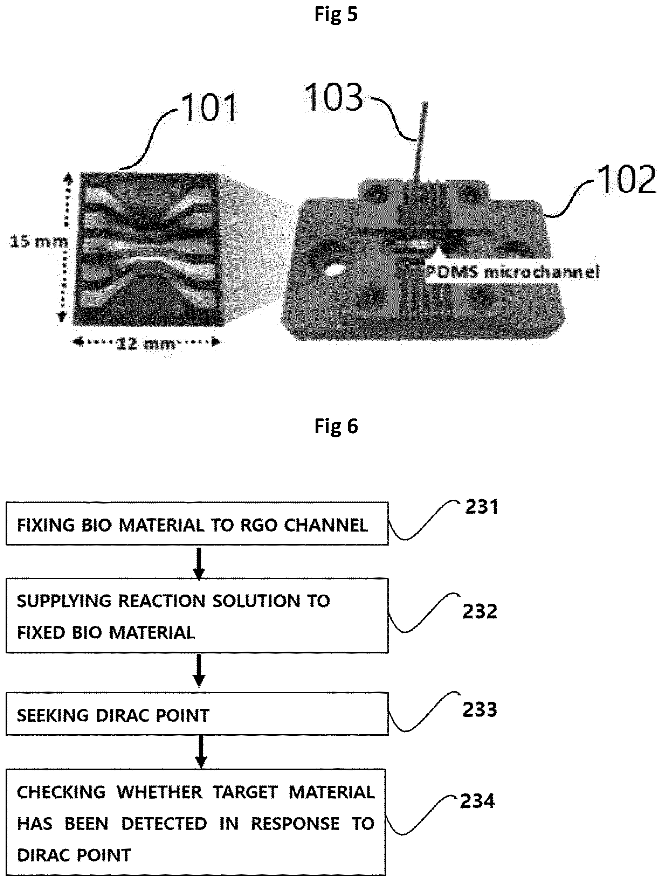

FIG. 5 is an example of a bio sensor (101) manufactured through the first to seventh steps.

The bio sensor (101) may be used by being mounted on a test jig, and a test may be realized in liquid phase by allowing a gate to be driven when a reference electrode (103) is directly contacted to the reaction solution through the PDMS chip.

That is, the reaction solution may be infused into the RGO FET type bio sensor coupled with the PDMS chip through the seventh step (217), and the bio sensor may be used as a gate for driving.

Referring to FIG. 6, a method for detecting a bio material will be described using the RGO-based bio sensor.

First, with reference to the RGO-based bio sensor (100) described by referring to FIG.1, or to the RGO-based bio sensor manufactured by the method for manufacturing an RGO-based bio sensor according to the present invention, a bio material may be fixed to the RGO channel (231). Here, the bio material means a bio material that functions as a linker for detecting a target material.

Furthermore, a reaction solution is supplied (232) to the bio material fixed at the '231' step. Although the supply of reaction solution at the '232' step is realized through a micro channel formed on the PDMS chip, and the reaction solution is supplied to the RGO channel, the reaction solution is prevented from flowing to the outside due to tight adherence between the passivation layer and the PDMS chip to thereby disallow being contacted to the drain electrode and the source electrode. Now, a Dirac point may be sought by measuring a current flowing between the drain electrode and the source electrode and a gate voltage (233).

At this time, the gate voltage may be directly applied to the reaction solution while being contacted to the RGO channel. Furthermore, whether to detect a target material and concentration may be determined (234) in response to the Dirac point sought from the '233' step.

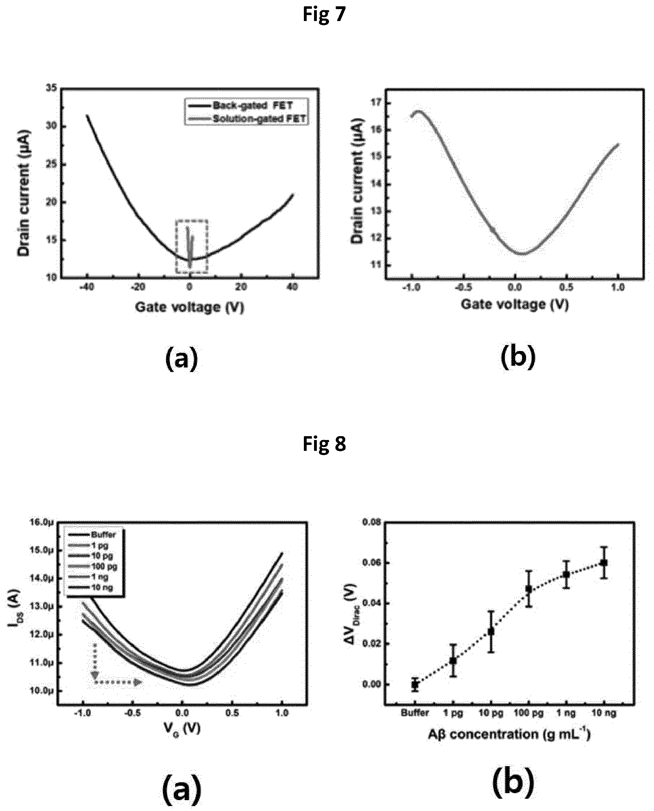

As shown in the example of FIG. 7, because the graphene is a conductive material having zero (0) in the band gap, it may show an ambipolar characteristic. That is, when field effect is applied, the graphene comes to possess a Dirac point, which is a charge neutral point. Because the said Dirac point takes a shape of being increased or decreased by the biochemical reaction generated from a graphene surface, the biochemical reaction may be determined based on a Dirac point (V_{Dirac}) when applied as a bio sensor. Meantime, when the conventional high-k material is used for a gate structure, a high gate voltage and source-drain voltage are required for driving (7a). However, a low gate voltage and source-drain voltage are required during the liquid phase driving (operation) as in the present invention (7b).

For example, when the RGO film is operated while in a liquid phase in an FET structure having an electric driving layer according to the present invention, a 'V' typed driving shape may be shown in "0.1V_{SD}, V_{G} = ±1V".

However, in case of the conventional FET, it is known that a smooth driving can be realized in "5V_{SD}, V_{G} = ±30V". This is because the electric conductivity of liquid is excellent when compared to high-k, which is due to the fact that the field effect phenomenon of RGO channel by the gate bias is smoothly generated under a low voltage environment.

As is evidenced in FIG. 7, a 'V' shaped lowest point (Dirac point), which is a standard for determining a biochemical change, can be observed in detail to allow having a higher resolution.

As a result, power consumption of bio sensor can be remarkably reduced and sensitivity based on binding affinity can be enhanced by applying a voltage directly to the reaction solution including a target material.

Now, an experiment will be explained in which a specific detection relative to a target material is performed by fixing a particular bio material to the RGO channel of the bio sensor according to the present invention.

FIG. 8 is an example of an experiment for quantitatively detecting Aβ protein, which is a bio marker, for diagnosing an Alzheimer's disease.

In order to perform a selective detection of Aβ protein, 6E10 antibody was fixated on a surface of the RGO channel, Aβ protein solution having 1 pg/mL ~ 1 ng/mL concentration was infused into the PDMS chip to induce a specific reaction.

As shown in FIG. 8a, it could be ascertained that there was a change in Dirac point, based on which, a quantitation curve as illustrated in FIG. 8b could be exhibited.

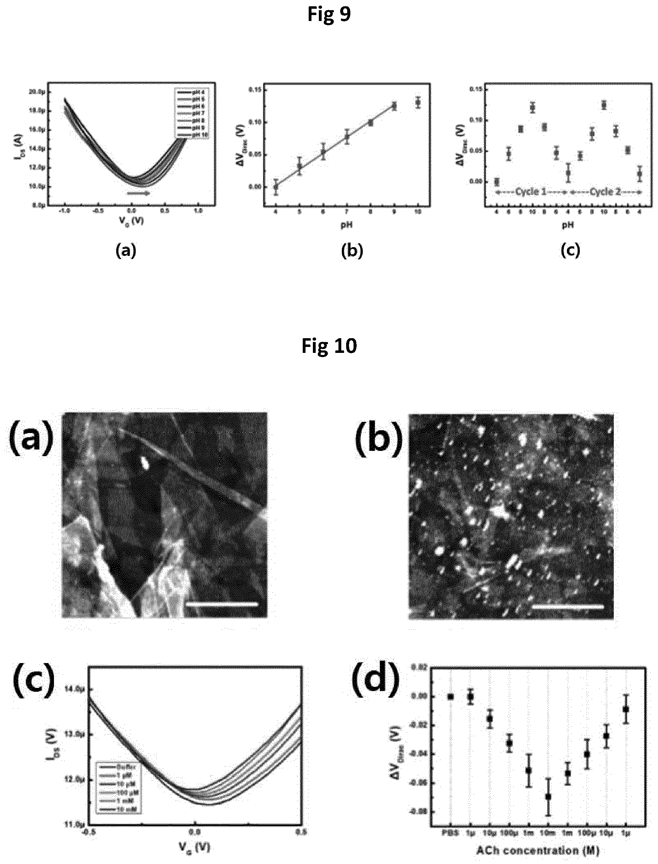

FIG. 9 illustrates an ion sensitivity of bio sensor from which reaction solutions having pH 4, 6, 8, 10 are infused to apply a field effect, and reaction reproducibility in response to change in H+ ion concentration could be observed through two cycles.

Furthermore, in order to perform an enzyme reaction detection, Acetycholinesterase (AChE) was fixated on the bio sensor, and Acetylcholine (ACh) was able to be quantitatively detected.

FIG. 10a shows a graphene surface with no treatment, while FIG.10b illustrates a surface of an RGO channel fixed with AChE using enzyme reaction detection experiment using noncovalent bond. FIG. 10c shows a change in Dirac point, and FIG. 10d shows a graph regarding AChE enzyme reaction.

The dynamic range of Ach was from 1µM to 10mM, and reproducibility could be ascertained on the reaction concentration as in the Ph change. Because hydrogen ion is generated through AChE enzyme reaction, through which changes are generated on an ion concentration near graphene surface where reaction occurs, reaction could be observed through the same mechanism as that of the result which had observed the above Ph change.

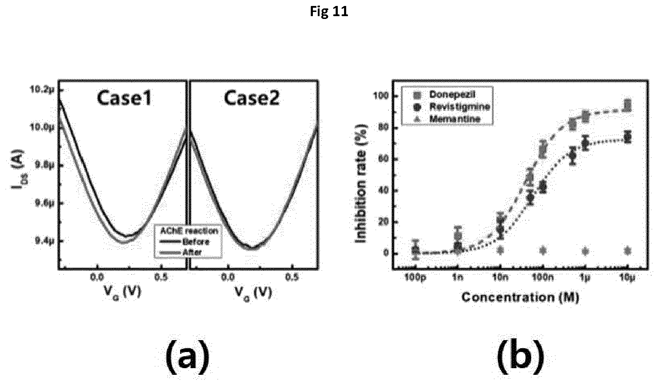

FIG. 11 is an example of drug-effect observation experiment using AChE inhibitor, where drug reaction related to AD symptom relief was observed.

In general, the symptoms of cognitive impairment of degenerative brain disease including AD is known to be generated from abnormal concentration of neurotransmitter like Ach. Therefore, treatment and remedy that alleviate the symptoms are commonly used by adjusting the neurotransmitter concentration within a body of an AD patient through AChE inhibitor. A normal AChE activity inhibiting reaction was observed through donepezil and rivastigmine which are representative AChE inhibitors.

Furthermore, a negative control experiment was performed using memantine that is used as inhibitor of different neurotransmitter enzyme within a body albeit not being reactive with AchE.

As explained above, the RGO channel of RGO-based FET type bio sensor is simultaneously utilized as electrodynamic layer and biochemical surfaction. Devices could be electrically driven within liquid by inducing field effect while fixing the bio material using a linker of noncovalent bond onto an RGO channel surface and applying a gate voltage to the reaction solution.

While the present invention has been described with reference to the particular illustrative embodiments, it is not to be restricted by the embodiments but only by the appended claims.

### [Description of Reference Numerals]

100: RGO-based bio sensor
110: substrate
121: source electrode
122: drain electrode
123: gate electrode
130: RGO channel
141: passivation layer
150: PDMS chip
151: micro channel

## Claims

1. A method for manufacturing a Reduced Graphene Oxide, RGO,-based bio sensor (100), the method comprising:
forming a source electrode (121) and a drain electrode (122) on a substrate (110) (first step);
forming an RGO layer on the substrate formed with the source electrode and the drain electrode (second step);
forming a first protective layer on the RGO layer (third step);
forming an RGO channel (130) between the source electrode and the drain electrode by performing a patterning process (fourth step);
forming a passivation layer (141) on the RGO channel, the source electrode and the drain electrode (fifth step); and
removing the first protective layer (sixth step), wherein:
the substrate (110) is an Si/SiO₂ substrate, and the second step includes:
fixing a 5% 3-aminopropyltriethoxysilane, APTES, onto the substrate;
depositing on the substrate, through a spin coating method, a solution obtained by diluting graphene oxide, GO, in distilled water to a concentration of 3mg/mL; and
exposing the deposited GO thin film to hydroiodic acid, HI, vapor at 80°C for 3 hours to cause a reduction reaction,
wherein the spin coating is performed for 30 seconds at 4,000 rpm.

2. The method of claim 1, wherein each of the source electrode and the drain electrode is formed with Cr/Au with a thickness of 20nm/100nm, and the first protective layer is formed with Al2O3 with a thickness of 50nm.

3. The method of claim 2, wherein the first protective layer in the sixth step is removed under an environment of wet etchant being heated at 180°C.

4. The method of claim 1, wherein the fourth step includes patterning the RGO channel on the first protective layer using photoresist and processing by dry etching method.

5. The method of claim 1, wherein the passivation layer is formed with a SU-8 photoresist.

6. The method of any one of the preceding claims, comprising: attaching, on the passivation layer, a polydimethylsiloxane, PDMS, chip (150) for supplying reaction solution to the RGO channel.

## Patentansprüche

1. Verfahren zur Herstellung eines Biosensors (100) auf Basis von reduziertem Graphenoxid, RGO, wobei das Verfahren umfasst:
Bilden einer Quellelektrode (121) und einer Drainelektrode (122) auf einem Substrat (110) (erster Schritt);
Bilden einer RGO-Schicht auf dem mit der Quellelektrode und der Drainelektrode gebildeten Substrat (zweiter Schritt);
Bilden einer ersten Schutzschicht auf der RGO-Schicht (dritter Schritt);
Bilden eines RGO-Kanals (130) zwischen der Quellelektrode und der Drainelektrode durch Ausführen eines Strukturierungsprozesses (vierter Schritt);
Bilden einer Passivierungsschicht (141) auf dem RGO-Kanal, der Quellelektrode und der Drainelektrode (fünfter Schritt); und
Entfernen der ersten Schutzschicht (sechster Schritt), wobei:
das Substrat (110) ein Si/SiO2-Substrat ist und der zweite Schritt beinhaltet:
Fixieren eines 5% 3-Aminopropyltriethoxysilans, APTES, auf das Substrat,
Abscheiden auf dem Substrat, durch ein Schleuderbeschichtungsverfahren, einer Lösung, die durch Verdünnen von Graphenoxid, GO, in destilliertem Wasser auf eine Konzentration von 3mg/mL erhalten wird, und
Aussetzen des abgeschiedenen GO-Dünnfilms an Jodwasserstoffsäure-, HI-, Dampf bei 80 °C für 3 Stunden, um eine Reduktionsreaktion zu bewirken,
wobei das Schleuderbeschichten für 30 Sekunden bei 4.000 rpm durchgeführt wird.

2. Verfahren nach Anspruch 1, wobei jede der Quellelektrode und der Drainelektrode mit Cr/Au mit einer Dicke von 20nm/100nm gebildet wird und die erste Schutzschicht mit Al2O3 mit einer Dicke von 50nm gebildet wird.

3. Verfahren nach Anspruch 2, wobei die erste Schutzschicht im sechsten Schritt in einer Umgebung aus Nassätzmittel, das auf 180 °C erhitzt wird, entfernt wird.

4. Verfahren nach Anspruch 1, wobei der vierte Schritt das Strukturieren des RGO-Kanals auf der ersten Schutzschicht unter Verwendung von Photoresist und das Verarbeiten durch Trockenätzverfahren beinhaltet.

5. Verfahren nach Anspruch 1, wobei die Passivierungsschicht mit einem SU-8-Photoresist gebildet wird.

6. Verfahren nach einem der vorhergehenden Ansprüche, umfassend: Befestigen, auf der Passivierungsschicht, eines Polydimethylsiloxan-, PDMS-, Chips (150) zum Versorgen des RGO-Kanals mit Reaktionslösung.

## Revendications

1. Procédé permettant la fabrication d'un biocapteur à base d'oxyde de graphène réduit, RGO, (100) le procédé comprenant :
la formation d'une électrode source (121) et d'une électrode drain (122) sur un substrat (110) (première étape) :
la formation d'une couche RGO sur le substrat formée avec l'électrode source et l'électrode drain (deuxième étape) ;
la formation d'une première couche protectrice sur la couche RGO (troisième étape) ;
la formation d'un canal RGO (130) entre l'électrode source et l'électrode drain par la réalisation d'un processus de structuration (quatrième étape) ;
la formation d'une couche de passivation (141) sur le canal RGO, l'électrode source et l'électrode drain (cinquième étape) ; et
le retrait de la première couche protectrice (sixième étape),
ledit substrat (110) étant un substrat Si/SiO2, et ladite seconde étape comprenant :
la fixation d'un 3-aminopropyltriéthoxysilane, APTES, à 5 % sur le substrat ;
le dépôt sur le substrat, par un procédé d'enduction centrifuge, une solution obtenue par dilution de l'oxyde de graphène, GO, dans de l'eau distillée à une concentration de 3 mg/mL ; et
l'exposition du film mince GO déposé à de la vapeur d'acide iodhydrique, HI, à 80°C pendant 3 heures pour entraîner une réaction de réduction,
ladite enduction centrifuge étant réalisée pendant 30 secondes à 4 000 tr/min.

2. Procédé selon la revendication 1, chacune de l'électrode source et de l'électrode drain étant formée de Cr/Au avec une épaisseur de 20 nm/100 nm, et ladite première couche protectrice étant formée d'Al2O3 avec une épaisseur de 50 nm.

3. Procédé selon la revendication 2, ladite première couche protectrice de la sixième étape étant retirée dans un environnement d'agent de gravure humide chauffé à 180°C.

4. Procédé selon la revendication 1, ladite quatrième étape comprenant la structuration du canal RGO sur la première couche protectrice à l'aide d'une résine photosensible et d'un traitement par un procédé de gravure sèche.

5. Procédé selon la revendication 1, ladite couche de passivation étant formée avec une résine photosensible SU-8.

6. Procédé selon l'une quelconque des revendications précédentes, comprenant : la fixation, sur la couche de passivation, d'une puce de polydiméthylsiloxane, PDMS (150) pour fournir la solution de réaction au canal RGO.
